(19) Europäisches Patentamt
European Patent Office
Office européen des brevets

(11) Veröffentlichungsnummer: **0 135 806**
**A2**

(12) **EUROPÄISCHE PATENTANMELDUNG**

(21) Anmeldenummer: **84110032.4**

(22) Anmeldetag: **23.08.84**

(51) Int. Cl.⁴: **C 07 C 50/36**, C 07 D 317/26, C 07 C 69/95

(30) Priorität: **30.08.83 FR 8313877**

(43) Veröffentlichungstag der Anmeldung: **03.04.85 Patentblatt 85/14**

(84) Benannte Vertragsstaaten: **AT BE CH DE GB IT LI LU NL SE**

(71) Anmelder: **LABORATOIRES HOECHST S.A., Tour Roussel Nobel 3, Avenue du Général de Gaulle, F-92800 Puteaux (FR)**

(72) Erfinder: **Gesson, Jean-Pierre, La Germonière Montamisé, F-86360 Chasseneuil du Poitou (FR)**
Erfinder: **Mondon, Martine, Appartement 529 rue du Pontreau 9, F-86000 Poitiers (FR)**
Erfinder: **Abdallah, Awad Abdallah, Soug El Shedra House Nr. 1-1/677, Omdurman (SD)**
Erfinder: **Jacquesy, Jean-Claude, rue du Planty 46, F-86180 Bruxerolles (FR)**
Erfinder: **Petit geb. Monpontet, Patricia, Place de Fontevrault 1-47, F-86000 Poitiers (FR)**

(74) Vertreter: **Meyer-Dulheuer, Karl-Hermann, Dr. et al, HOECHST Aktiengesellschaft Zentrale Patentabteilung Postfach 80 03 20, D-6230 Frankfurt/Main 80 (DE)**

(54) **Neue Aglykone zur Herstellung von Antibiotika und Antitumormitteln aus der Klasse der Anthracycline und Verfahren zu deren Herstellung.**

(57) Vorliegende Erfindung betrifft neue Aglykone, die der allgemeinen Formel IV

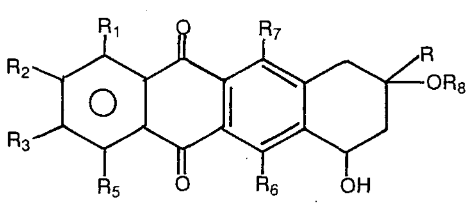

IV

entsprechen, worin

$R_1$, $R_2$, $R_3$ und $R_5$, die gleich oder verschieden sein können, für ein Wasserstoffatom oder eine OH-, O-Metall- oder $OR_4$-Gruppe,

wobei $R_4$ eine gegebenenfalls substituierte $C_1$–$C_6$-Alkylgruppe bedeutet,

R für ein Wasserstoffatom oder eine $COCH_2R'$-Gruppe, wobei R' Wasserstoff oder eine Alkyl-, Hydroxyl-, Alkoxyl- oder Arylgruppe bedeutet,

$R_6$ und $R_7$, die stets verschieden sind, entweder für ein Wasserstoffatom oder eine OH-Gruppe und

$R_8$ für ein Wasserstoffatom oder eine $CH_3CO$-Gruppe stehen,

zur Verwendung bei der Herstellung von Antibiotika und Antitumormitteln.

LABORATOIRES HOECHST S.A. HOE 83/S 027 Dr.HA

Neue Aglykone zur Herstellung von Antibiotika und Antitumormitteln aus der Klasse der Anthracycline und Verfahren zu deren Herstellung

Vorliegende Erfindung betrifft zur Herstellung von Antibiotika und Antitumormitteln aus der Klasse der Anthracycline verwendbare neue Aglykone sowie Verfahren zu deren Herstellung.

Die Anthracycline entsprechen der nachfolgenden allgemeinen Formel (I)

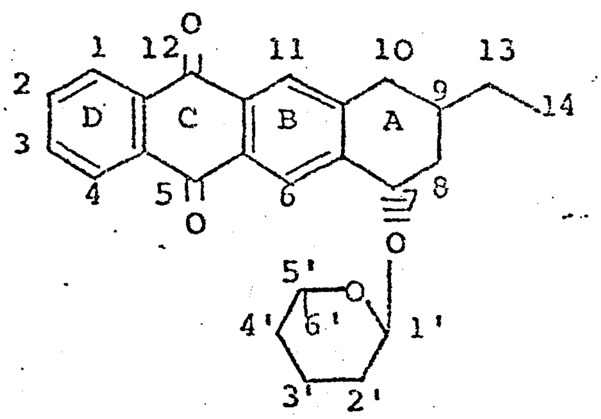

(I)

und stellen eine wichtige Gruppe von Antibiotika und Antitumormitteln dar. Gewisse Mitglieder dieser Gruppe (wie beispielsweise Daunorubicin und Doxorubicin) stehen erfolgreich im klinischen Einsatz zur Behandlung verschiedener Krebsformen (akute Leukämien, Brustkrebs, Krebs in den Harnwegen, Hodgkin-Krankheit usw.).

Andererseits wird ihre bemerkenswerte cytotoxische Wirksamkeit durch schädliche Nebenwirkungen eingeschränkt, insbesondere eine erhebliche Herztoxizität. Diese ist mit der verwendeten Dosis des Antibiotikums verknüpft und erfordert irreversible Unterbrechung der Behandlung über einem gewissen Schwellenwert.

Im Lauf der letzten Jahre wurden zahlreiche Anstrengungen unternommen, die therapeutische Breite dieser Verbindungen zu verbessern; dabei dürfte eine Verringerung der Herztoxizität wichtiger sein als eine Erhöhung ihrer gewichtsmässi-

gen Wirksamkeit.

Die durchgeführten Abänderungen beziehen sich auf das Aglykon und/oder den Zucker durch Totalsynthese, Biosynthese, oder auch Halbsynthese.

Abänderungen wurden in allen Ringen (A, B, C, D) und auch am Zucker durchgeführt. Im allgemeinen werden die Produkte je nach der Substitution im Ring B in verschiedene Kategorien eingeteilt:

I - 6- und 11-Dihydroxylderivate (dazu gehören das Daunorubicin - Verbindung II - und Doxorubicin - Verbindung III).

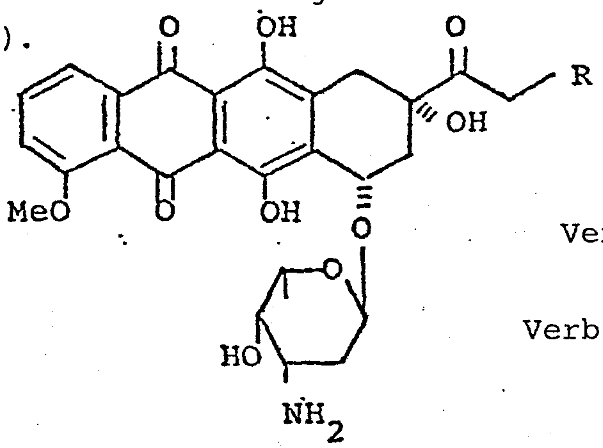

Verbindung (II): R = H

Verbindung (III): R = OH

Daunorubicin (II): gleichzeitig in Italien (Farmitalia) und Frankreich (Rhône-Poulenc) entdeckt

Doxorubicin (III): 1969 aus Streptomyces peucetius var. caesius isoliert (Farmitalia).

II - 6-Monohydroxylderivate (11-Desoxyreihe)

III - 11-Monohydroxylderivate (6-Desoxyreihe)

I - Beispiele für 6- und 11-Dihydroxylderivate

1 - Abänderungsversuche am Ring D

Die in 4-Stellung desmethoxylierten Analoga der Verbindungen (II) und (III) wurden hergestellt; diese besitzen eine bessere therapeutische Breite als die natürlichen Verbindungen (F. Arcamone, Anticancer Antibiotics, Academic Press, 1981, Kapitel 7). Ferner wurden verschiedene Substituenten eingeführt: Cl und $CH_3$ in 1- und 4-Stellung oder Cl, $CH_3$ oder Benzo in 2- und 3-Stellung (vgl. F. Arcamone et al., Experientia 34, 1255, 1978).

Der Benzolring D wurde durch einen schwefelhaltigen aromatischen Heterocyclus ersetzt (A.S. Kende und H. Newman, Europäische Patentanmeldung 17 469, 15. Oktober 1980).

2 - Abänderungsversuche am Ring C

Nur wenige Arbeiten in dieser Richtung, doch sei die Herstellung des 5-Imino-daunorubicins genannt (L. Tong, D.W. Henry und E.M. Acton, J. Med. Chem., 22, 36 (1979)).

3 - Abänderungsversuche am Ring A

Zahlreiche Analoge wurden hergestellt, meistens halbsynthetisch durch Umwandlung natürlicher Produkte: 10- oder 8-$OCH_3$-Derivate, 9-Desoxyverbindungen (mit oder ohne Δ-9-Doppelbindung), Verbindungen mit modifizierten Seitenketten (9-Desacyl), reduzierte Produkte, Derivate der Carbonylgruppe in 13-Stellung (vgl. F. Arcamone, Anticancer Antibiotics, a.a.O.).

4 - Abänderungsversuche am Zucker

Abänderungen wurden in sämtlichen Stellungen (1', 2', 3', 4', 5', 6') des Pyranosylrings durchgeführt, wobei die Antitumoraktivität in vivo häufig beibehalten oder sogar verbessert wurde.

II - Beispiele für 6-Monohydroxyderivate (11-Desoxyreihe)

Im Jahr 1980 erhielt Farmitalia 11-Desoxy-dauno- und -doxorubicin durch Fermentation. Diese besitzen eine ähnliche Antitumoraktivität in vivo wie die Verbindungen (II) und (III) bei höheren Dosierungen aber mit niedrigerer Toxozität.

Zahlreiche Totalsynthesen der entsprechenden Aglykone wurden beschrieben (vgl. insbesondere J.P. Gesson und M. Mondon, Chem. Comm., 1982, 421), und ebenso für 4-Desmethoxy-11-desoxydaunorubicin und -doxorubicin (H. Umezawa

und Mitarbeiter, J. of Antibiotics 33, 1581, (1980).

Es sei bemerkt, dass es auch natürliche Verbindungen aus der Anthracyclinklasse gibt, die in 11-Stellung kein Hydroxyl tragen: Aclacinomycine, Pyrromycine, Cinerubine usw.

III - <u>Beispiele für 11-Derivate (6-Desoxyreihe)</u>

Es sind gewisse natürliche Anthracycline bekannt, die kein Hydroxyl in 6-Stellung tragen: $\alpha$-Citromycin und $\alpha_2$-Rhodomycin.

Dagegen wurden bisher keine 6-Desoxy-dauno- oder doxorubicine isoliert oder synthetisiert.

In einer neueren Veröffentlichung beschreiben F. Arcamone und Mitarbeiter (J. Org. Chem., <u>48</u>, 405, 1983) die Synthese von 4-Desmethoxy-6-desoxydaunorubicin, dessen erste Untersuchungen in vitro (HeLa-Zellen) eine ähnliche Cytotoxizität wie Daunorubicin aufzeigen.

Die Wirkungsweise all dieser Moleküle ist wahrscheinlich komplex. Es wird angenommen, dass es sich dabei um eine Einlagerung in die DNS-Kette handelt, wobei dieser Vorgang durch die teilweise Planarität des tetracylischen Systems begünstigt wird.

Danach erfolgt eine Reduktion des Moleküls, die als Zwischenprodukt zu einem Ionenradikal führt, das der Ausgangspunkt der Bildung toxischer Körper (z.B. $HO^\bullet$, $H_2O_2$) im Gewebebereich sein könnte, und schliesslich die Bildung einer reduzierten Form des Chinonsystems. Diese Form wäre für die Abspaltung des Zuckers und die Bildung freien Aglykons (ohne Sauerstoffunktion in 7-Stellung) verantwortlich.

Diese Ueberlegungen führten Hansch und Mitarbeiter (Il Farmaco 35, 12, 965 (1980)), dazu, die Einführung von kleinen Donorgruppierungen (OH, $OCH_3$) in 2- oder 3-Stellung vorzuschlagen.

Man kann sich in der Tat vorstellen, dass die Einführung verschiedener Substituenten in den Ring D durch Modifizierung des Redoxpotentials des Chinonsystems diese verschiedenen Reaktionen beschränken und zu Verbindungen mit überlegener therapeutischer Breite führen könnte. Dies bedeutet die Synthese neuer Verbindungen durch zahlreiche Forscher sowie eine Untersuchung möglicher Beziehungen zwischen dem Redoxpotential und/oder der Antitumoraktivität und/oder der Toxizität.

Einerseits durch eine Studie und Untersuchung der von verschiedenen Forschern erhaltenen Ergebnisse und andererseits durch Berücksichtigung der Tatsache, dass

1. bei ansonsten gleichen Faktoren gewisse natürliche Verbindungen der 11-Desoxyreihe mit Hydroxyl in 1-Stellung eine stärkere Antitumoraktivität als in dieser Stellung nicht hydroxylierte Verbindungen aufweisen (z.B. die Aktivitäten gegenüber Cinerubin-A und Aclacinomycin-A; Lit. S.T. Crook und S.D. Reich, Anthracyline, Academic Press 1980, Kapitel 6) und
2. der reduktive Abspaltungsvorgang am L-Daunosamin sicherlich durch die Gegenwart des Phenols in peri-Stellung sowie analog der raschen und selektiven Hydrogenolyse einer Benzylalkoholfunktion in 10-Stellung des α-Citromycinons beschleunigt wird,

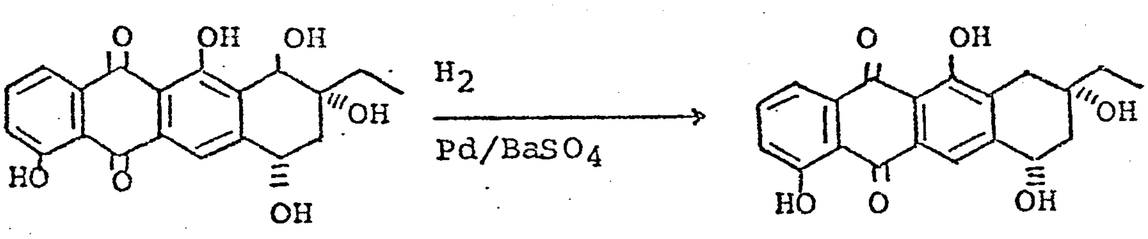


α-Citromycinon γ-Citromycinon

ist es der Anmelderin gelungen, eine ganze Reihe von Verbindungen - Aglykonen - zu synthetisieren, die hochaktive Medikamente geringer Toxizität liefern können.

Gegenstand vorliegender Erfindung sind neue, zur Herstellung von Antibiotika und Antitumormitteln verwendbare Aglykone, welche dadurch gekennzeichnet sind, dass sie der nachfolgenden allgemeinen Formel IV entsprechen:

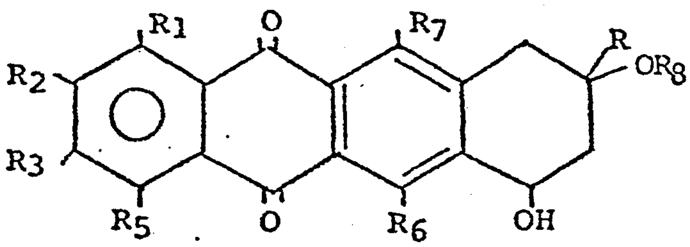
(IV)

worin

$R_1$, $R_2$, $R_3$ und $R_5$, die gleich oder verschieden sein können, für ein Wasserstoffatom oder eine OH-, O-Metall- oder $OR_4$-Gruppe, wobei $R_4$ eine gegebenenfalls substituierte $C_1$-$C_6$-Alkylgruppe bedeutet,

R für ein Wasserstoffatom oder eine $COCH_2R'$-Gruppe, wobei R' Wasserstoff oder eine Alkyl-, Hydroxyl-, Alkoxyl- oder Arylgruppe bedeutet,

$R_6$ und $R_7$, die stets verschieden sind, entweder für ein Wasserstoffatom oder eine OH-Gruppe und

$R_8$ für ein Wasserstoffatom oder eine $CH_3CO$-Gruppe stehen,

mit der Massgabe, dass

1) $R_1$, $R_2$ und $R_3$ niemals gleichzeitig Wasserstoff bedeuten, wenn $R_7$ und $R_8$ ein Wasserstoffatom und R die $COCH_2R'$-Gruppe darstellen, und

2) $R_5$ von Wasserstoff und $OCH_3$ verschieden ist, wenn $R_1$, $R_2$, $R_3$ und $R_6$ für Wasserstoff stehen.

Erfindungsgemäss stehen die beiden OH-Gruppen im Ring A in cis- oder trans-Stellung zueinander.

Gemäss einer vorteilhaften Ausbildung des Erfindungsgegenstands stellen die erhaltenen Produkte 11-Desoxyaglykone dar.

Gemäss einer weiteren vorteilhaften Ausbildung des Erfindungsgegenstands stellen die erhaltenen Produkte 6-Desoxyaglykone dar.

Erfindungsgemäss werden die 11-Desoxyaglykone aus der das Aglykon mit $R_1$ = OH, $R_2$ = $R_3$ = $R_5$ = H, $R_6$ = OH, R = $COCH_3$ und $R_8$ = H,

das Aglykon mit $R_1 = R_3 = R_5 = H$, $R_2 = OCH_3$, $R_6 = OH$, $R = COCH_3$ und $R_8 = H$,
das Aglykon mir $R_1 = R_2 = R_5 = H$, $R_3 = OCH_3$, $R_6 = OH$, $R = COCH_3$ und $R_8 = H$ und
das Aglykon mit $R_1 = R_2 = R_3 = R_5 = R = H$, $R_6 = OH$ und $R_8 = COCH_3$
umfassenden Gruppe ausgewählt.

Gemäss einer vorteilhaften Ausführung des Erfindungsgegenstands entspricht das 6-Desoxyaglykon dem Produkt mit $R_1 = R_2 = R_3 = R_6 = H$, $R_7 = OH$, $R_5 = OH$ und $R = COCH_3$.

Gegenstand vorliegender Erfindung ist weiterhin ein Verfahren zur Herstellung der erfindungsgemässen 11-Desoxyaglykone, welches durch die folgenden Stufen gekennzeichnet ist:
<u>1. Stufe</u>: <u>Herstellung des Enoläthers der allgemeinen Formel V</u>

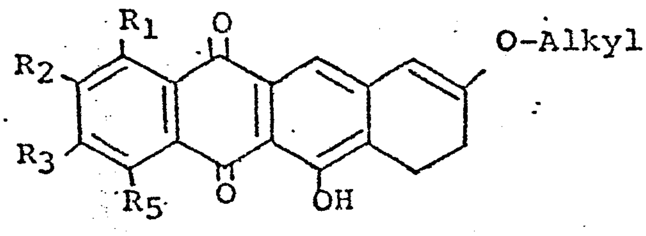
(V)

worin
$R_1$, $R_2$, $R_3$ und $R_5$ die oben angegebenen Bedeutungen haben,

durch Einwirkung eines halogenierten Naphthochinons der allgemeinen Formel VI auf ein Dien der allgemeinen Formel VII

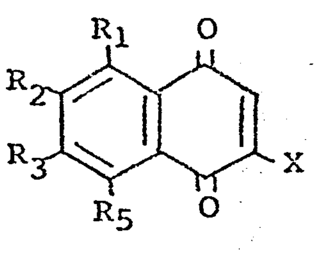
+
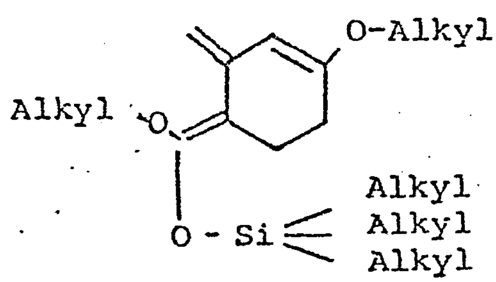

(VI) (VII)

worin
$R_1$, $R_2$, $R_3$ und $R_5$ die oben angegebenen Bedeutungen haben und
X für ein Halogenatom steht;

2. Stufe: Hydrolyse des Enoläthers V zum entsprechenden Keton der allgemeinen Formel VIII

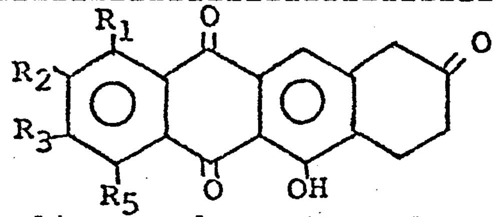

(VIII)

3. Stufe: Aethinylierung des Ketons VIII zum entsprechenden Produkt der Formel IX

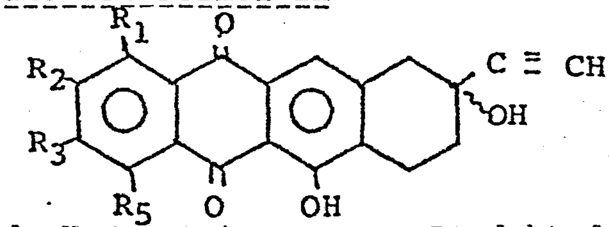

(IX)

und nachfolgende Hydratisierung zum Produkt der allgemeinen Formel X

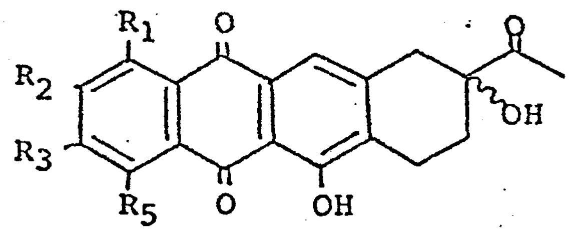

(X)

4. Stufe: Bromierung am Benzylkohlenstoff und nachfolgende basische Hydrolyse zum gewünschten Produkt.

Gegenstand vorliegender Erfindung ist weiterhin ein Verfahren zur Herstellung erfindungsgemässer 6-Desoxyaglykone, welches dadurch gekennzeichnet ist, dass man im Verlauf der 1. Stufe ein Dien der allgemeinen Formel XI

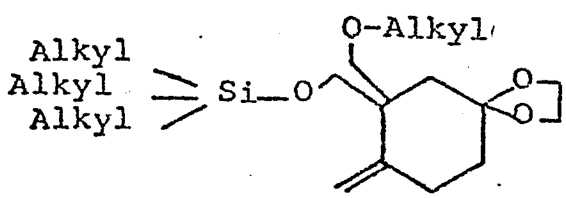

(XI)

einsetzt. Die übrigen Stufen sind dem für die 11-Desoxyderivate beschriebenen Verfahren ähnlich.

Ausser den obigen Ausführungsformen umfasst die Erfindung noch weitere Ausführungsformen, die aus der nachfolgenden Beschreibung, die sich auf Herstellungsbeispiele für erfindungsgemässe Produkte bezieht, ersichtlich sind. Dabei versteht es sich jedoch, dass diese Beispiele nur der Erläuterung des Erfindungsgegenstands dienen und keine einschränkende Bedeutung besitzen.

BEISPIEL 1 - Herstellung des 5-Hydroxy-8-methoxy-3,4-dihydro-1H-2,6,11-naphthacentrions

Zu einer Lösung von 3-Chlor-6-methoxy-1,4-naphthochinon (1,9 g; 8,6 mMol) in Tetrahydrofuran (THF) (20 ml) gibt man tropfenweise bei 0°C das Ketenacetal VII (2,5 g; 8,9 mMol), gelöst in THF (3 ml), und rührt das Reaktionsgemisch über Nacht bei Raumtemperatur. Nach Abdampfen des Lösungsmittels gibt man den erhaltenen Feststoff langsam bei -40°C zu einer Lösung von $SbF_5$ (5 ml) in HF (25 ml) in einem Teflonkolben. Man hält das Gemisch 20 Minuten bei -40°C und giesst es dann langsam auf 500 ml Wasser/Eisgemisch. Nach Extraktion mit Methylenchlorid wäscht man die organische Phase mit Wasser, trocknet über Natriumsulfat und dampft dann ein. Das tetracyclische Keton wird durch Chromatographie unter Druck (Laufmittel: $CH_2Cl_2$) gereinigt (1,11 g; 40%).

BEISPIEL 2 - Herstellung des (±)-2,5-Dihydroxy-2-äthinyl-8-methoxy-1,2,3,4-tetrahydro-6,11-napthacendions

Man lässt mittels Durchleiten durch eine erste, mit Eis gekühlte Falle und dann durch eine konzentrierte Schwefelsäure enthaltende Falle gereinigtes Acetylen 1 Stunde lang durch 40 ml wasserfreies THF perlen. Dann versetzt man tropfenweise mit Aethylmagnesiumbromid (40 ml, 1m in THF, 40 mMol). Nach der Zugabe dampft man bei vermindertem Druck ein und nimmt in 80 ml über $P_2O_5$ destilliertem Methylenchlorid ($CH_2Cl_2$) auf. Diese Lösung wird auf -40°C gekühlt und tropfenweise mit dem in Beispiel 1 hergestellten, in $CH_2Cl_2$ (150 ml) gelösten Keton (386 mg; 1,2 mMol) versetzt. Das Reaktionsgemisch wird 1 Stunde zwischen -40°C und -20°C weitergerührt, anschliessend mittels 0,1n-Salzsäurelösung hydrolysiert und dann auf übliche Weise mit Methylenchlorid extrahiert. Durch Chromatographie unter Druck ($CH_2Cl_2$)

lässt sich das nicht umgesetzte Ausgangsketon (148 mg, 38%) von dem entstandenen Aethinylcarbinol (165 mg, 43%) trennen.

BEISPIEL 3 - Herstellung des (±)-4-Desmethoxy-7,11-didesoxy-3-methoxy-daunomycinons

Man behandelt Amberlitharz IRN 77 mit Quecksilberoxyd HgO auf folgende Weise: das Harz (50 g) wird in verdünnter $H_2SO_4$-Lösung suspendiert und dann mehrmals durch Dekantieren mit Wasser gewaschen. Dann gibt man eine Lösung von Quecksilberoxyd (0,5 g) in 1 Liter verdünnter Schwefelsäure zu dem Harz. Das Harz wird erneut mit Wasser gewaschen und dann unter vermindertem Druck getrocknet.

Man erhitzt ein Gemisch aus wie in Beispiel 2 angegeben hergestelltem Aethinylcarbinol (140 mg; 0,4 mMol) und HgObehandeltem Harz (2 g) in 200 ml Aethanol und 40 ml Wasser 16 Stunden lang zum Rückfluss. Man kühlt das Reaktionsgemisch ab und filtriert, extrahiert das Filtrat mit Methylenchlorid, wäscht die organische Phase mit Wasser und trocknet über Natriumsulfat. Verdampfung des Methylenchlorids unter vermindertem Druck ergibt ein Hydroxyketon (145 mg; 98%), das ohne weitere Reinigung eingesetzt wird.

BEISPIEL 4 - Herstellung des (±)-4-Desmethoxy-11-desoxy-3-methoxy-daunomycinons

Eine Lösung von Hydroxyketon X (100 mg; 0,27 mMol) und Epoxycyclohexan (132 mg; 1,35 mMol) in 250 ml $CCl_4$ und 50 ml $CH_2Cl_2$ wird unter Stickstoff mit einer 60 W-Lampe bestrahlt, während man tropfenweise eine Bromlösung in $CCl_4$ (0,36 ml 0,1m-Lösung mit 50 ml $CCl_4$ verdünnt) dazugibt. Nach der Zugabe wird die Lösung 30 Minuten weitergerührt und dann unter vermindertem Druck eingedampft. Man nimmt das Reaktionsgemisch in 60 ml Aceton auf und versetzt mit einer aus Calciumoxyd erhaltenen Calciumhydroxydlösung (48 mg CaO, 0,82 mMol in 50 ml Wasser). Das violette Reaktionsgemisch wird 30 Minuten weitergerührt, dann mit gesättigter Oxalsäurelösung neutralisiert und in üblicher Weise

mit Methylenchlorid extrahiert. Durch Chromatographie unter Druck ($CH_2Cl_2$/MeOH = 99/1) lässt sich das (±)-4-Desmethoxy-11-desoxy-3-methoxy-daunomycinon (37,2 mg; 36%) von (±)-7-Epi-4-desmethoxy-11-desoxy-3-methoxy-daunomycinon (43,8 mg; 42,5%) trennen.

BEISPIEL 5 - Herstellung des (±)-2-(1,1-Aethylendioxy-äthyl)-2,7,12-trihydroxy-1,2,3,4-tetrahydro-6,11-naphthacendions

Man erhitzt eine Lösung von (±)-2-Acetyl-2,7,12-trihydroxy-1,2,3,4-tetrahydro-6,11-naphthacendion (740 mg; 2,1 mMol) und Aethylenglykol (280 mg; 4,4 mMol) in Gegenwart einer katalytischen Menge para-Toluolsulfonsäure in 400 ml Benzol 12 Stunden lang zum Rückfluss in einem 500 ml-Kolben, der mit einem Dean- und Stark-Aufsatz versehen ist, damit das gebildete Wasser als Azeotrop mit Benzol entfernt werden kann.

Nach Waschen mit Wasser und Trocknen über Natriumsulfat wird die organische Phase eingedampft, was das Dioxolan (816 mg; 98%) ergibt.

BEISPIEL 6 - Herstellung des (±)-4-Desmethoxy-6-desoxy-4-hydroxy-daunomycinons

Man versetzt eine Lösung des wie in Beispiel 5 angegeben erhaltenen Dioxolans (260 mg; 0,66 mMol), Epoxycyclohexan (324 mg; 3,3mMol) und Azobis-isobutyronitril (30 mg; 0,18 mMol) in 400 ml $CCl_4$ bei 45°C mit Bromlösung (8 ml einer 0,1m-Lösung; 0,8 mMol). Man hält 3 Stunden bei dieser Temperatur und dampft dann bei vermindertem Druck ein. Das Reaktionsgemisch wird in einer Lösung von 0,5n-Natronlauge in THF (1 zu 1) aufgenommen und über Nacht bei Raumtemperatur weitergerührt. Nach Neutralisation mit 0,1n-HCl und üblicher Extraktion mit Methylenchlorid wird das Rohprodukt in einer auf 0°C gekühlten Lösung von $CF_3COOH$ (20 ml) in $H_2O$ (4 ml) aufgenommen und 4 Stunden bei dieser Temperatur weitergerührt. Man giesst die Lösung auf 200 ml Eis/Wassergemisch und extrahiert mit Methylenchlorid.

Das erhaltene Aglykon (66 mg; 28%)

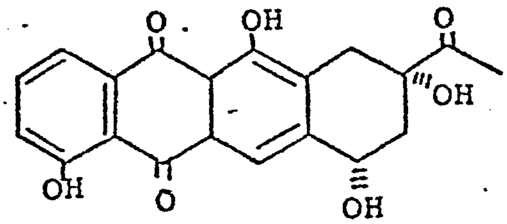

wird durch Dünnschichtchromatographie ($CH_2Cl_2$/MeOH = 98/2) gereinigt.

BEISPIEL 7 - Herstellung des (±)-4-Desmethoxy-9-desacetyl-7,11-didesoxy-daunomycinons

Man versetzt eine Lösung des tetracyclischen Ketons VIII (3 g; 10,2 mMol) in 900 ml THF und 90 ml Wasser langsam mit Natrium-cyanborhydrid (6,2 g; 102 mMol). Während der Zugabe hält man das Reaktionsgemisch durch Zugabe verdünnter 1n-Salzsäurelösung auf pH 2-3. Die Lösung wird noch 30 Minuten weitergerührt und dann mit Methylenchlorid extrahiert. Die mit Wasser gewaschene und über Natriumsulfat getrocknete organische Phase wird unter vermindertem Druck eingedampft, was den entsprechenden Alkohol (2,95 g; 98%) liefert, der keine weitere Reinigung erfordert.

BEISPIEL 8 - Herstellung des (±)-9-O-Acetyl-4-desmethoxy-9-desacetyl-7,11-didesoxy-daunomycinons

Man rührt eine Lösung des wie in Beispiel 7 angegebenen Alkohols (470 mg; 1,6 mMol) in 15 ml Essigsäureanhydrid und 30 ml Pyridin 16 Stunden lang bei -10°C. Man giesst das Reaktionsgemisch auf Eis/Wasser und extrahiert mit Methylenchlorid. Die mit 1n-Salzsäurelösung und dann mit Wasser gewaschene organische Phase wird über Natriumsulfat getrocknet, und das Lösungsmittel wird unter vermindertem Druck abgedampft. Durch Chromatographie unter Druck (Laufmittel: $CH_2Cl_2$) des Rückstands lässt sich das Acetat (430 mg; 80%) von dem gebildeten Diacetat (54 mg; 9%) und Ausgangsalkohol (24 mg; 5%) trennen.

BEISPIEL 9 - Herstellung des (±)-9-O-Acetyl-4-desmethoxy-9-desacetyl-11-desoxy-daunomycinons

Eine Lösung der vorhergehenden Verbindung (145 mg; 0,43 mMol) in 700 ml $CCl_4$ wird unter Stickstoff in Gegenwart von Epoxycyclohexan (216 mg; 2,2mMol) mit einer 60 W-Lampe bestrahlt, während man tropfenweise eine Bromlösung in $CCl_4$ (4,8 ml 0,1m-Lösung mit 70 ml $CCl_4$ verdünnt) dazugibt. Nach der Zugabe wird die Lösung 30 Minuten weitergerührt und dann unter vermindertem Druck eingedampft. Man nimmt das Reaktionsgemisch in 100 ml THF und 50 ml 0,1n-Salzsäure auf, rührt 1 Stunde 30 Minuten bei gewöhnlicher Tempeatur und extrahiert in üblicher Weise mit Methylenchlorid.

Durch Chromatographie unter Druck (Laufmittel: $CH_2Cl_2$) des Rohprodukts lässt sich das (±)-9-O-Acetyl-4-desmethoxy-9-desacetyl-11-desoxy-daunomycinon (43 mg; 28%) vom (±)-7-Epi-9-O-acetyl-4-desmethoxy-9-desacetyl-11-desoxy-daunomycinon (74 mg; 49%) trennen.

EIGENSCHAFTEN
DER ERHALTENEN PRODUKTE

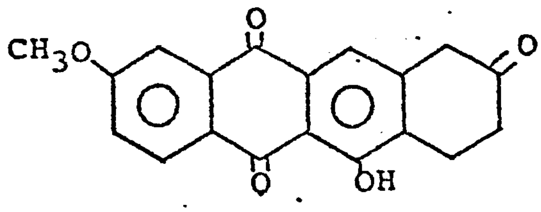

5-Hydroxy-9-methoxy-3,4-dihydro-1H-2,6,11-naphthacentrion

Ausbeute: 40%.

Schmelzpunkt: 232-234°C.

Massenspektrum: m/z = 322 (96%), 294 (75%), 293 (67%), 280 (100%).

IR (KBr-Scheibe): 2960, 1710, 1670, 1630, 1590, 1570, 1490, 1470, 1430, 1420 1370, 1345, 1310, 1295, 1280, 1230, 1170, 1110, 1085, 1060, 1030, 1000 und 960 $cm^{-1}$.

NMR ($CDCl_3$): 2,6 ppm (Triplett, J = 7Hz, 2H); 3,27 (Triplett, J = 7Hz, 2H); 3,68 (Singulett, 2H); 3,98 (Singulett, 3H); 7,23 (doppeltes Dublett, J = 7Hz und 2Hz, 1H); 7,53 (Singulett, 1H); 7,68 (Dublett, J = 2Hz, 1H); 8,20 (Dublett, J = 7Hz, 1H); 13,1 (Singulett, 1H).

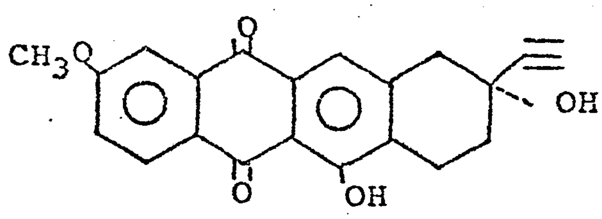

(±)-2,5-Dihydroxy-2-äthinyl-9-methoxy-1,2,3,4-tetrahydro-6,11-naphthacendion

Ausbeute: 50%.

Zersetzungspunkt 230°C.

Massenspektrum m/z = 348 (57%), 330 (70%), 322 (57%), 280 (100%).

IR (KBr-Scheibe): 3530, 3440, 3290, 3240, 2960, 1670, 1630, 1600, 1470, 1430, 1380, 1370, 1340, 1295, 1270, 1230, 1200, 1090, 1020, 1010, 980, 940, 850, 820, 800 und 750 $cm^{-1}$.

NMR ($CDCl_3$): 2,16 ppm (Triplett, J = 7 Hz, 2H); 2,47 (Singulett, 1H); 3,22 (Singulett 2H); 3,03 (Triplett, J = 7 Hz, 2H); 3,99 (Singulett, 3H); 7,24 (doppeltes Dublett, J = 7 Hz, und 2Hz, 1H); 7,54 (Singulett, 1H); 7,69 (Dublett, J = 2Hz, 1H); 8,20 (Dublett, J = 7Hz, 1H); 13,15 Singulett, 1H).

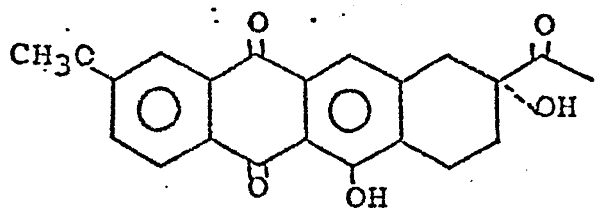

($\pm$)-2-Acetyl-2,5-dihydroxy-9-methoxy-1,2,3,4-tetrahydro-6,11-naphthacendion

($\pm$)-4-Desmethoxy-7,11-didesoxy-2-methoxydaunomycinon

Ausbeute: 98%.

Schmelzpunkt: 244-246 $^{o}$C.

Massenspektrum: m/z = 366 (10%), 348 (6%), 323 (100%), 305 (33%), 293 (25%), 280 (18%).

IR (KBr-Scheibe): 3600 (fein), 2965, 1715, 1670, 1630, 1595, 1570, 1500, 1470, 1460, 1450, 1425, 1410, 1390, 1360, 1350, 1340, 1300, 1270, 1250, 1230, 1200, 1175, 1105, 1090, 1060, 1040, 1025, 990, 960, 940, 920, 895, 880, 850, 815, 795, 780, 770, 750, 720, 680, 630 und 610 $cm^{-1}$.

NMR ($CDCl_3$): 2,02 ppm (Multiplett, 2H); 2,37 (Singulett, 3H); 3,12(Multiplett, 4H); 3,99 (Singulett, 3H); 7,25 (doppeltes Dublett, J = 7 Hz und 2Hz, 1H); 7,58 (Singulett, 1H); 7,71 (Dublett, J = 2 Hz, 1H); 8,20 (Dublett, J = 7 Hz, 1H); 13,18 (Singulett, 1H).

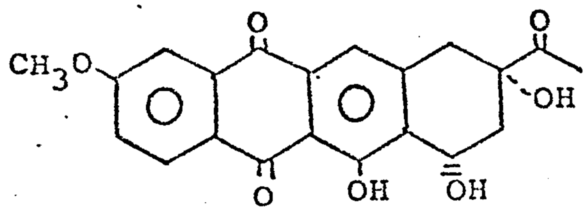

(±)-2-Acetyl-9-methoxy-2α,4α,5-trihydroxy-1,2,3,4-tetrahydro-6,11-naphthacendion

(±)-4-Desmethoxy-11-desoxy-2-methoxy-daunomycinon

Ausbeute: 35%
Schmelzpunkt = 220-223°C
Massenspektrum: m/z = 382 (15%), 346 (100%), 331 (66%), 321 (49%), 293 (30%), 247 (22%).
IR (KBr-Scheibe): 3250 (breit), 2960, 1715, 1670, 1630, 1595, 1500, 1475, 1440, 1390, 1370, 1350, 1340, 1320, 1300, 1270, 1230, 1215, 1205, 1175, 1125, 1105, 1085, 1060, 1040, 1030, 1005, 985, 930, 910, 900, 870, 850, 830, 810, 785, 775, 750, 740, 730 und 635 $cm^{-1}$.
NMR ($CDCl_3$): 2,28 ppm (2H); 2,42 (Singulett, 3H); 3,06 (Singulett, 1H); 3,18 (Singulett, 1H); 4,00 (Singulett, 3H); 4,58 (1H); 5,30 (Multiplett, Halbwertsbreite = 8,5Hz, 1H); 7,26 (doppeltes Dublett, J = 7 Hz und 2Hz, 1H); 7,63 (Singulett, 1H); 7,70 (Dublett, J = 2 Hz, 1H); 8,26 (Dublett, J = 7 Hz, 1H); 13,43 (Singulett, 1H).

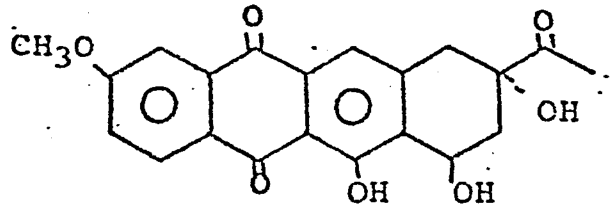

(±)-2-Acetyl-9-methoxy-2α,4β,5-trihydroxy-1,2,3,4-tetrahydro-6,11-naphthacendion

(±)-7-Epi-4-desmethoxy-11-desoxy-2-methoxy-daunomycinon

Ausbeute: 34%

Schmelzpunkt = 203-204°C

Massenspektrum m/z = 382 (15%), 346 (65%), 321 (100%), 293 (58%), 268 (20%).

IR (KBr-Scheibe): 3250 (breit), 2970, 2930, 1710, 1670, 1630, 1595, 1570, 1500, 1470, 1425, 1390, 1360, 1340, 1300, 1280, 1230, 1200, 1100, 1070, 1025, 985 und 820 $cm^{-1}$.

NMR ($CDCl_3$): 2,39 ppm (Singulett, 3H); 3,99 (Singulett, 3H), 5,41 (breites Triplett, Halbwertsbreite = 20 Hz); 7,25 (doppeltes Dublett, J = 7 Hz und 2 Hz, 1H); 7,59 (Singulett, 1H); 7,72 (Dublett, J = 2 Hz, 1H); 8,25 (Dublett, J = 7Hz, 1H); 13,68 (Singulett, 1H).

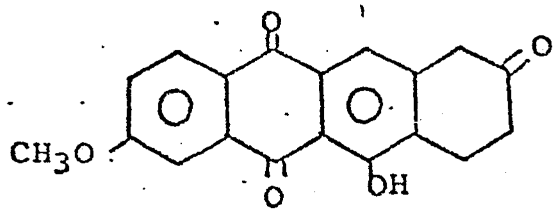

5-Hydroxy-8-methoxy-3,4-dihydro-1H-2,6,11-naphthacentrion

Ausbeute: 40%

Schmelzpunkt = 248-250°C

Massenspektrum: m/z = 322 (73%), 294 (39%), 293 (24%), 280 (100%).

IR (KBr-Scheibe): 2970, 1710, 1660, 1620, 1590, 1570, 1490, 1455, 1450, 1430, 1415, 1375, 1340, 1290, 1260, 1230, 1190, 1150, 1075, 1020, 1000, 915, 875, 860, 845, 820, 770, 750, 740, 680, 650 und 630 $cm^{-1}$.

NMR ($CDCl_3$): 2,6 ppm (Triplett, J = 7 Hz, 2H); 3,25 (Triplett, J = 7 Hz, 2H); 3,68 (Singulett, 2H); 3,98 (Singulett, 3H); 7,25 (doppeltes Dublett, J = 7 Hz und 2 Hz, 1H); 7,53 (Singulett, 1H); 7,70 (Dublett, J = 7 Hz, 1H); 8,20 (Dublett, J = 7 Hz, 1H); 12,93 (Singulett, 1H).

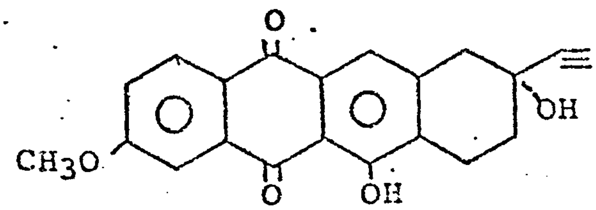

$(\pm)$-2,5-Dihydroxy-2-äthinyl-8-methoxy-1,2,3,4-tetrahydro-6,11-naphthacendion

Ausbeute: 43%
Schmelzpunkt = 230-231°C
Massenspektrum m/z = 348 (73%), 330 (99%), 280 (100%).
IR (KBr-Scheibe): 3600 (breit), 3260, 2975, 2930, 1670, 1630, 1595, 1570, 1500, 1470, 1430, 1420, 1390, 1350, 1330, 1320, 1300, 1280, 1240, 1170, 1100, 1090, 1050, 1025, 1010, 990, 970, 930, 910, 860, 850, 840, 810, 785, 770, 755 und 690 $cm^{-1}$.
NMR ($CDCl_3$): 2,17 ppm (Triplett, J = 7 Hz, 2H); 2,49 (Singulett, 1H); 3,22 (Singulett, 2H); 3,08 (Triplett, J = 7 Hz, 2H); 3,99 (Singulett, 3H); 7,27 (doppeltes Dublett, J = 7 Hz und 2 Hz, 1H); 7,54 (Singulett, 1H); 7,22 (Dublett, J = 2Hz, 1H); 8,22 (Dublett, J = 7 Hz, 1H); 12,97 (Singulett, 1H).

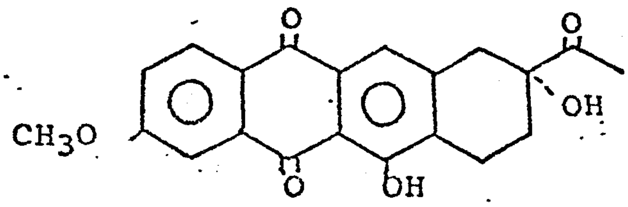

(±)-2-Acetyl-2,5-dihydroxy-8-methoxy-1,2,3,4-tetrahydro-6,11-naphthacendion

(±)-4-Desmethoxy-7,11-didesoxy-3-methoxy-daunomycinon

Ausbeute: 98%
Schmelzpunkt = 208-210°C
Massenspektrum: m/z = 366 (8%), 343 (4%), 323 (100%), 305 (25%), 293 (16%), 280 (12%).
IR (KBr-Scheibe): 3500, 3060, 2960, 1700, 1660, 1630, 1590, 1560, 1540, 1480, 1420, 1380, 1350, 1340, 1330, 1290, 1275, 1240, 1200, 1160, 1105, 1080, 1030, 1000, 990, 980, 950, 925, 910, 895, 860, 850, 825, 810, 800, 770, 750, 720, 710 und 630 $cm^{-1}$.
NMR ($CDCl_3$): 2,02 (Triplett, 2H); 2,37 (Singulett, 3H); 3,07 (Multiplett, 4H); 3,99 (Singulett, 3H); 7,26 (doppeltes Dublett, J = 7 Hz und 2 Hz, 1H); 7,54 (Singulett, 1H); 7,72 (Dublett, J = 2 Hz, 1H); 8,23 (Dublett, J = 7 Hz, 1H); 12,99 (Singulett, 1H).

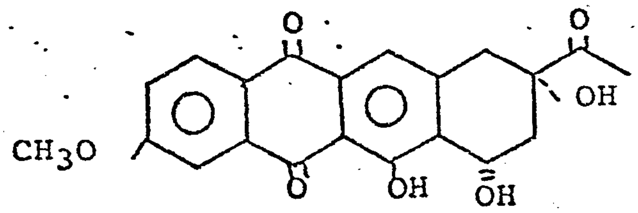

(±)-2-Acetyl-8-methoxy-2α,4α,5-trihydroxy-1,2,3,4-tetrahydro-6,11-naphthacendion

(±)-4-Desmethoxy-11-desoxy-3-methoxy-daunomycinon

Ausbeute: 36%
Schmelzpunkt = 222-223 °C
Massenspektrum: m/z = 382 (15%), 346 (100%), 331 (66%), 321 (49%), 293 (30%), 247 (22%).

IR (KBr-Scheibe): 3540, 2980, 2970, 1700, 1660, 1630, 1590, 1570, 1450, 1420, 1380, 1350, 1330, 1310, 1300, 1280, 1250, 1240, 1200, 1160, 1120, 1105, 1100, 1080, 1020, 1010, 990, 970, 930, 900, 880, 840, 820, 800, 770, 760 und 630 $cm^{-1}$.

NMR ($CDCl_3$): 2,28 (Multiplett, 2H); 2,41 (Singulett, 3H); 4,00 (Singulett, 3H); 5,23 (Multiplett, Halbwertsbreite = 10 Hz, 1H); 7,25 (doppeltes Dublett, J = 7 Hz und 2 Hz, 1H); 7,61 (Singulett, 1H); 7,70 (Dublett, J = 2Hz, 1H); 8,26 (Dublett, J = 7 Hz, 1H); 13,19 (Singulett, 1H).

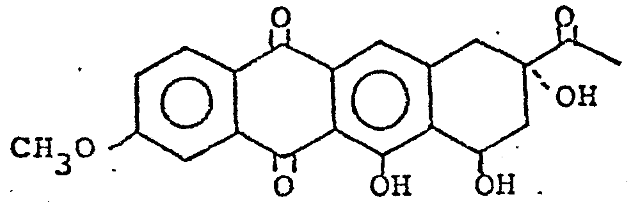

(±)-2-Acetyl-8-methoxy-2α,4β,5-trihydroxy-1,2,3,4-tetrahydro-6,11-naphthacendion

(±)-7-Epi-4-desmethoxy-11-desoxy-3-methoxy-daunomycinon

Ausbeute: 42,5%
Massenspektrum: m/z = 382 (11%), 346 (100%), 321 (97%), 293 (50%), 268 (16%), 247 (21%).
IR (KBr-Scheibe): 3490 (breit), 2960, 2930, 1720, 1670, 1640, 1600, 1430, 1390, 1360, 1340, 1290, 1240, 1170, 1105, 1095, 1040, 1020 und 910 $cm^{-1}$.
NMR ($CDCl_3$): 2,40 ppm (Singulett, 3H); 4,00 (Singulett, 3H); 5,43 (breites Triplett, Halbwertsbreite = 20 Hz, 1H); 7,25 (doppeltes Dublett, J = 2 Hz und 7 Hz, 1H); 7,60 (Singulett, 1H); 7,73 (Dublett, J = 2 Hz, 1H); 8,25 (Dublett, J = 7 Hz, 1H); 13,45 (Singulett, 1H).

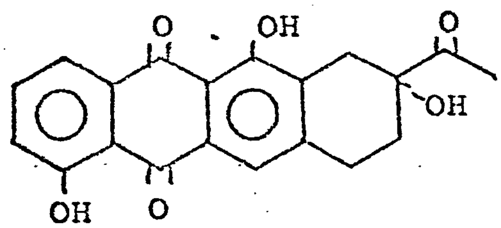

(±)-4-Desmethoxy-6,7-didesoxy-4-hydroxy-daunomycinon

(±)-2-Acetyl-2,7,12-trihydroxy-1,2,3,4-tetrahydro-6,11-naphthacendion

Ausbeute: 97%

Schmelzpunkt = 188-190°C

Massenspektrum: m/z = 352 (6%), 334 (20%), 309 (100%), 291 (24%), 280 (20%), 279 (23%), 266 32%).

IR (KBr-Scheibe): 3505, 2940, 2860, 1700, 1630, 1590, 1490, 1460, 1430, 1400, 1340, 1290, 1230, 1190, 1170, 1120, 1090, 1060, 1050, 965, 930, 915, 835, 815, 790, 730 und 700 $cm^{-1}$.

NMR ($CDCl_3$): 2,39 (Singulett, 3H); 3,08 (Multiplett, 4h); 7,2-7,8 (Multiplett, 4H); 12,70 (Singulett, 1H); 13,09 (Singulett, 1H).

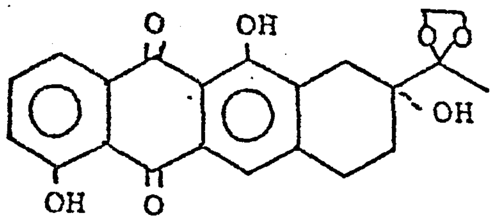

(+)-2-(1,1-Aethylendioxy-äthyl)-2,7,12-trihydroxy-1,2,3,4-tetrahydro-6,11-naphthacendion

Ausbeute: 98%

Schmelzpunkt = 216-217°C

Massenspektrum : m/z = 396 (0,5%), 308 (85%), 266 (100%).

IR (KBr-Scheibe): 3440, 3000, 2950, 1640, 1600, 1570, 1480, 1460, 1430, 1400, 1380, 1360, 1320, 1310, 1280, 1260, 1200, 1170, 1100, 1090, 1080, 1060, 1040, 970, 955, 930, 910, 890, 850, 840, 800, 780, 730, 700 und 650 $cm^{-1}$.

NMR ($CDCl_3$): 1,43 ppm (Singulett, 3H); 2,0 (Multiplett, 2H); 2,94 (Multiplett, 4H); 4,08 (Singulett, 4H); 7,5 (Multiplett, 4H); 12,73 (Singulett, 1H); 13,08 (Singulett, 1H).

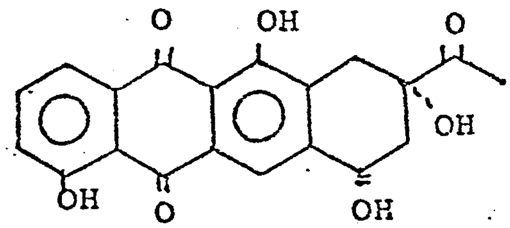

(±)-2-Acetyl-2α,4α,7,12-tetrahydroxy-1,2,3,4-tetrahydro-6,11-naphthacendion

(±)-4-Desmethoxy-6-desoxy-4-hydroxy-daunomycinon bzw. (±)-6-Desoxy-carminomycinon

Ausbeute: 28%

Schmelzpunkt = 219-221°C

Massenspektrum: m/z = 368 (8%), 350 (19%), 332 (32%), 307 (78%), 279 (100%).

IR (KBr-Scheibe): 3530, 3360, 2960, 2930, 1710, 1630, 1600, 1570, 1470, 1450, 1420, 1390, 1250, 1220, 1180, 1170, 1130, 1080, 1055, 1030, 950, 910, 900, 840 und 790 $cm^{-1}$.

NMR ($CDCl_3$): 2,3 ppm (Multiplett, 2H); 2,42 (Singulett, 3H); 3,07 (Singulett, 2H); 4,95 (Multiplett, Halbwertsbreite = 8,6 Hz, 1H); 7,25-8,04 (Multiplett, 4H); 12,71 (Singulett, 1H); 13,14 (1H).

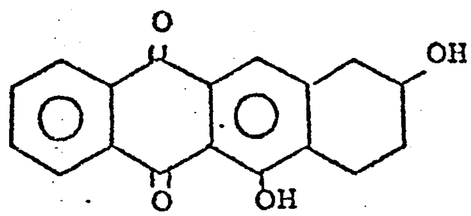

(±)-2,5-Dihydroxy-1,2,3,4-tetrahydro-6,11-naphthacendion

(±)-4-Desmethoxy-9-desacetyl-7,11-desoxy-daunomycinon

Ausbeute: 98%
Schmelzpunkt = 283-285°C
Massenspektrum: m/z = 294, 276
IR (KBr-Scheibe): 3400 (breit), 2960, 1660, 1630, 1590, 1470, 1410, 1380, 1360, 1330, 1320, 1270, 1250, 1200, 1160, 1090, 1060, 1050, 1000, 970, 920, 820, 800 und 710 $cm^{-1}$.

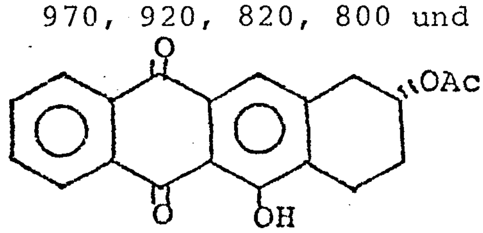

(±)-2-Acetoxy-5-hydroxy-1,2,3,4-tetrahydro-6,11-naphthacendion

(±)-9-O-Acetyl-4-desmethoxy-9-desacetyl-7,11-desoxy-daunomycinon

Ausbeute: 80%
Schmelzpunkt = 205-206°C
Massenspektrum: m/z = 336 (7%), 276 (100%).
IR (KBr-Scheibe): 3440 (breit), 2975, 1730, 1670, 1625, 1590, 1570, 1480, 1430, 1410, 1380, 1370, 1350, 1290, 1280, 1250, 1220, 1060, 1040, 970, 930, 790, 750 und 725 $cm^{-1}$.
NMR ($CDCl_3$): 2,0 ppm (Multiplett, 2H); 2,07 (Singulett, 3H); 2,9 (Multiplett, 4H); 5,23 (breites Triplett, Halbwertsbreite = 13 Hz, 1H); 7,4 (Singulett, 1H); 7,7 (Multiplett, 2H); 8,15 (Multiplett, 2H); 12,8 (Singulett, 1H).

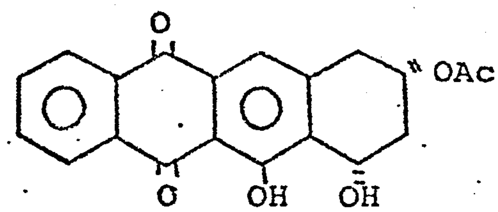

(±)-2α-Acetoxy-4α,5-dihydroxy-1,2,3,4-tetrahydro-6,11-naphthacendion

(±)-9-O-Acetyl-4-desmethoxy-9-desacetyl-11-desoxy-daunomycinon

Ausbeute: 28%

Schmelzpunkt = 182-183°C

Massenspektrum: m/z = 352 (1,5%), 292 (60%), 274 (100%).

IR (KBr-Scheibe): 3500, 2980, 2960, 1730, 1670, 1630, 1590, 1520, 1480, 1430, 1420, 1390, 1370, 1290, 1270, 1250, 1060, 1030, 970, 800, 790 und 720 $cm^{-1}$.

NMR ($CDCl_3$): 2,07 ppm (Singulett, 3H); 2,33 (Multiplett, 2H); 3,1 (Dublett, J = 4Hz, 2H); 3,67 (Dublett, J = 4 Hz, 1H); 5,23 (Multiplett, Halbwertsbreite = 16Hz, 2H); 7,5 (Singulett, 1H); 7,8 (Multiplett, 2H); 8,23 (Multiplett, 2H); 13,2 (Singulett, 1H).

 

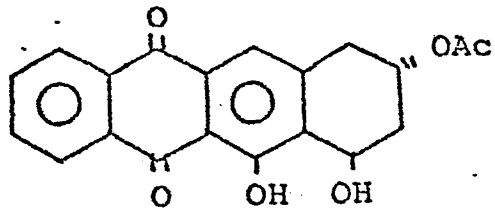

(±)-2-Acetoxy-4,5-dihydroxy-1,2,3,4-tetrahydro-6,11-naphthacendion

(±)-7-Epi-9-O-acetyl-4-desmethoxy-9-desacetyl-11-desoxy-daunomycinon

Ausbeute: 49%
Schmelzpunkt = 222-227°C (Zers.)
Massenspektrum: m/z = 352 (1%), 292 (18%), 274 (100%).
IR (KBr-Scheibe): 2980, 2960, 1720, 1670, 1630, 1590, 1570, 1480, 1450, 1420, 1390, 1365, 1355, 1330, 1290, 1280, 1250, 1240, 1100, 1070, 1060, 1040, 980, 950, 910, 830, 790 und 730 $cm^{-1}$.
NMR ($CDCl_3$): 2,07 ppm (Singulett, 3H); 5,36 (Multiplett, Halbwertsbreite = 11,5 Hz, 2H); 7,59 (Singulett, 1H); 7,80 (Multiplett, 2H); 8,25 (Multiplett, 2H); 13,37 (Singulett, 1H).

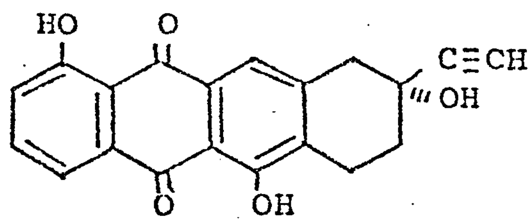

(±)-2-Aethinyl-2,5,10-trihydroxy-1,2,3,4-tetrahydro-6,11-naphthacendion

Ausbeute: 60%
Schmelzpunkt = 225-226°C
IR ($CH_2Cl_2$): 3420, 3300, 2960, 2940, 1640, 1610, 1580, 1485, 1475, 1460, 1430, 1395, 1340, 1320, 1270, 1205, 1195, 1165, 1150, 1095, 1060, 1050, 1030, 1000, 960, 915, 840 und 780 $cm^{-1}$.
Massenspektrum: m/z = 334 (12%), 333 (54%), 316 (45%), 307 (55%), 280 (39%), 266 (100%).

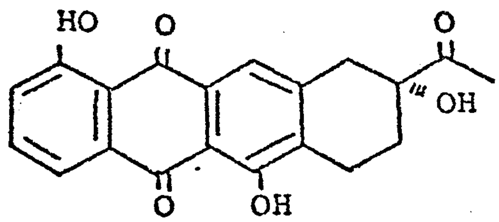

($\pm$)-2-Acetyl-2,5,10-trihydroxy-1,2,3,4-tetrahydro-6,11-naphthacendion

Ausbeute quantitativ

Schmelzpunkt = 226-228°C

Massenspektrum: m/z = 352 (26%), 309 (100%), 291 (42%), 279 (37%), 266 (18%), 205 (24%), 189 (24%), 164 (22%), 152 (25%), 121 (53%), 115 (41%), 93 (46%), 91 (46%).

IR (KBr): 3460, 2920, 1860, 1705, 1635, 1625, 1615, 1610, 1600, 1580, 1570, 1470, 1450, 1410, 1395, 1365, 1335, 1280, 1270, 1190, 1160, 1105, 1090, 1050, 970, 950, 905, 800 785 und 710 $cm^{-1}$.

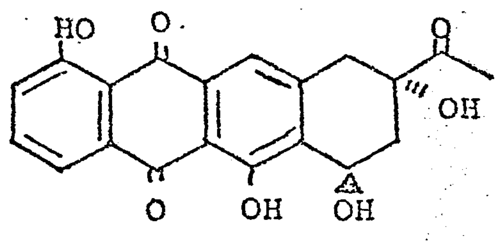

($\pm$)-2-Acetyl-2α,4α,5,10-tetrahydroxy-1,2,3,4-tetrahydro-6,11-naphthacendion

Schmelzpunkt = 209-211°C

IR (KBr): 3500, 3350, 2940, 1695, 1615, 1610, 1580, 1475, 1455, 1420, 1395, 1375, 1340, 1320, 1275, 1245, 1210, 1160, 1120, 1090, 1080, 1060, 1030, 975, 940, 905, 900, 840, 810, 800, 730 und 695 $cm^{-1}$.

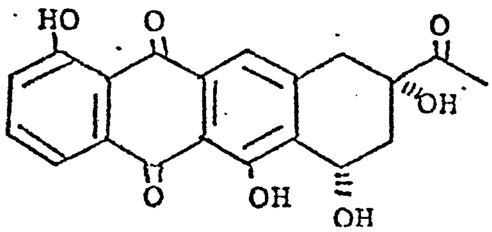

(±)-2-Acetyl-2α,4α,10-trihydroxy-1,2,3,4-tetrahydro-6,11-naphthacendion

= 4-Desmethoxy-11-desoxy-1-hydroxy-daunomycinon

Schmelzpunkt = 209-211 °C

Massenspektrum: m/z = 368 (11%), 332 (14%), 317 (11%), 307 (29%), 279 (18%), 205 (10%), 131 (32%), 119 (42%), 106 (69%), 105 (100%).

IR (KBr): 3580, 3520, 3490, 2930, 2860, 1720, 1635, 1620, 1590, 1490, 1470, 1435, 1400, 1385, 1350, 1290, 1275, 1220, 1175, 1125, 1120, 1080, 1070, 1040, 930 und 815 $cm^{-1}$.

Patentansprüche:

1. Neue Aglykone zur Herstellung von Antibiotika und Antitumormitteln, dadurch gekennzeichnet, dass sie der nachstehenden allgemeinen Formel IV entsprechen:

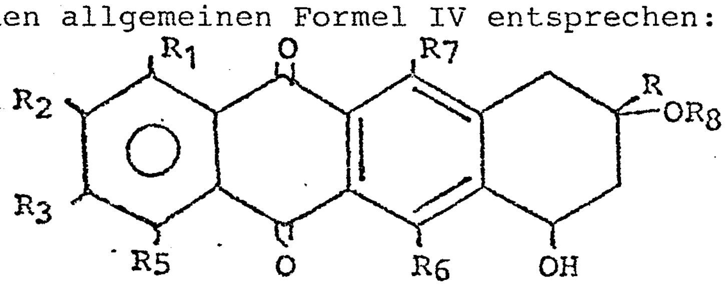

(IV)

worin

$R_1$, $R_2$, $R_3$ und $R_5$, die gleich oder verschieden sein können, für ein Wasserstoffatom oder eine OH-, OMetall- oder $OR_4$-Gruppe, wobei $R_4$ eine gegebenenfalls substituierte $C_1$-$C_6$-Alkylgruppe bedeutet,

R für ein Wasserstoffatom oder eine $COCH_2R'$-Gruppe, wobei R' Wasserstoff oder eine Alkyl-, Hydroxyl-, Alkoxyl- oder Arylgruppe bedeutet,

$R_6$ und $R_7$, die stets verschieden sind, entweder für ein Wasserstoffatom oder eine OH-Gruppe und

$R_8$ für ein Wasserstoffatom oder eine $CH_3CO$-Gruppe stehen,

mit der Massgabe, dass

1) $R_1$, $R_2$ und $R_3$ niemals gleichzeitig Wasserstoff bedeuten, wenn $R_7$ und $R_8$ ein Wasserstoffatom und R die $COCH_2R'$-Gruppe darstellen, und

2) $R_5$ von Wasserstoff und $OCH_3$ verschieden ist, wenn $R_1$, $R_2$, $R_3$ und $R_6$ für Wasserstoff stehen.

2. Aglykone nach Anspruch 1, dadurch gekennzeichnet, dass die beiden OH-Gruppen im Ring A in cis-Stellung zueinander stehen.

3. Aglykone nach Anspruch 1, dadurch gekennzeichnet, dass die beiden OH-Gruppen im Ring A in trans-Stellung zueinander stehen.

4. Aglykone nach einem der Ansprüche 1 bis 3, dadurch

gekennzeichnet, dass sie 11-Desoxyaglykone darstellen.

5. Aglykone nach einem der Ansprüche 1 bis 3, dadurch gekennzeichnet, dass sie 6-Desoxyaglykone darstellen.

6. Aglykone nach Anspruch 4, dadurch gekennzeichnet, dass sie aus der umfassenden Gruppe der Aglykone - das Aglykon mit $R_1$ = OH, $R_2 = R_3 = R_5$ = H, $R_6$ = OH, R = $COCH_3$ und $R_8$ = H, das Aglykon mit $R_1 = R_3 = R_5$ = H, $R_2 = OCH_3$, $R_6$ = OH, R = $COCH_3$ und $R_8$ = H, das Aglykon mit $R_1 = R_2 = R_5$ = H, $R_3 = OCH_3$; $R_6$ = OH, R = $COCH_3$ und $R_8$ = H und das Aglykon mit $R_1 = R_2 = R_3 = R_5$ = R = H, $R_6$ = OH und $R_8 = COCH_3$ ausgewählt sind.

7. Aglykone nach Anspruch 5, dadurch gekennzeichnet, dass sie dem Produkt entsprechen, worin $R_1 = R_2 = R_3 = R_6$ = H, $R_7$ = OH, $R_5$ = OH und R = $COCH_3$.

8. Verfahren zur Herstellung von erfindungsgemässen 11-Desoxyaglykonen, gekennzeichnet durch die folgenden Stufen:
1. Stufe: Herstellung des Enoläthers der allgemeinen Formel V

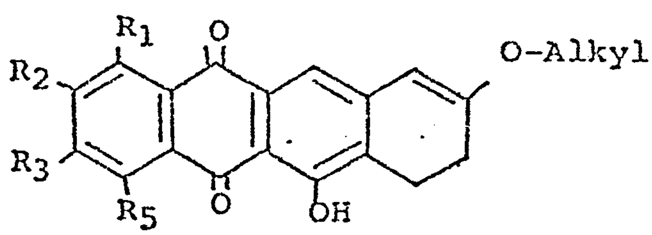

(V)

worin

$R_1$, $R_2$, $R_3$ und $R_5$ die oben angegebenen Bedeutungen haben,

durch Einwirkung eines halogenierten Naphthochinons der allgemeinen Formel VI auf ein Dien der allgemeinen Formel VII

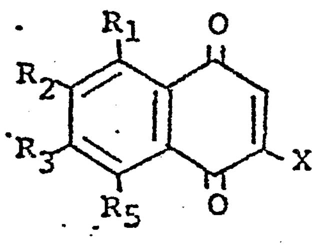 + 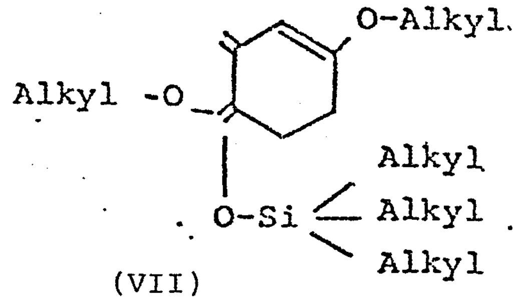

(VI) (VII)

worin

$R_1$, $R_2$, $R_3$ und $R_5$ die oben angegebenen Bedeutungen haben und

X für ein Halogenatom steht;

2. Stufe: Hydrolyse des Enoläthers V zum entsprechenden Keton der allgemeinen Formel VIII

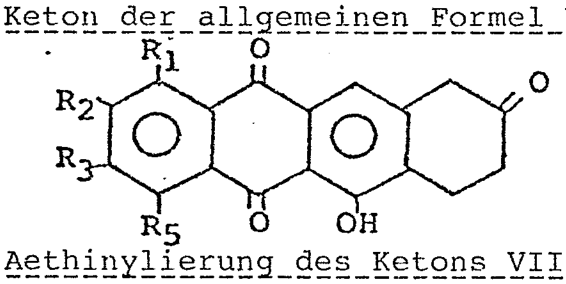

(VIII)

3. Stufe: Aethinylierung des Ketons VIII zum entsprechenden Produkt der Formel IX

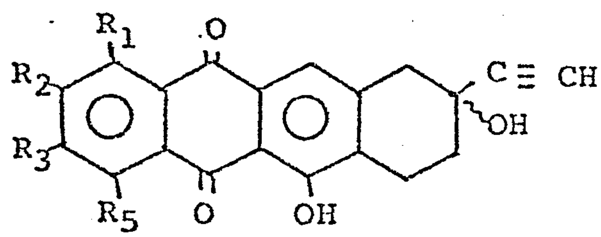

(IX)

und nachfolgende Hydratisierung zum Produkt der allgemeinen Formel X

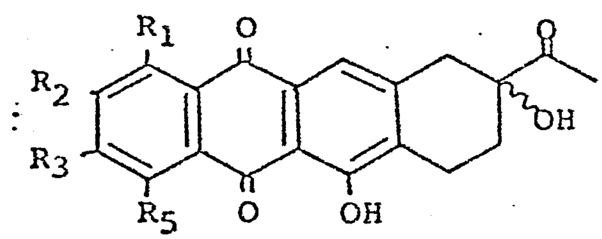

(X)

4. Stufe: Bromierung am Benzylkohlenstoff und nachfolgende basische Hydrolyse zum gewünschten Produkt.

9. Verfahren zur Herstellung von erfindungsgemässen 6-Desoxyaglykonen, dadurch gekennzeichnet, dass man im Ver-

lauf der 1. Stufe ein Dien der allgemeinen Formel XI

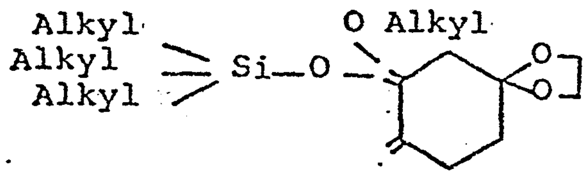

(XI)

einsetzt, wobei die übrigen Stufen dem in Anspruch 8 für die 11-Desoxyderivate beschriebenen Verfahren ähnlich sind.

10. Verbindung, deren einer Antipode der allgemeinen Formel XII

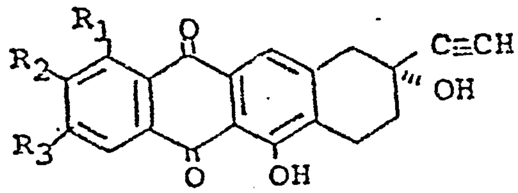

(XII)

entspricht, worin $R_1$, $R_2$ und $R_3$ die oben angegebenen Bedeutungen haben, als Zwischenprodukt zur Synthese der Aglykone nach Anspruch 1.

11. Verbindung, deren einer Antipode der allgemeinen Formel XIII

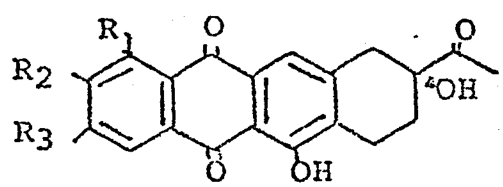

(XIII)

entspricht, worin $R_1$, $R_2$ und $R_5$ die oben angegebenen Bedeutungen haben, als Zwischenprodukt zur Synthese der Aglykone nach Anspruch 1.

12. Verbindung, deren einer Antipode der allgemeinen Formel XIV

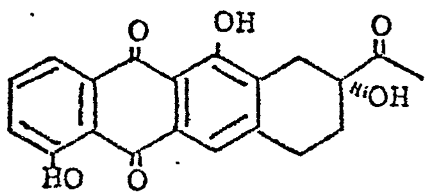

(XIV)

entspricht, als Zwischenprodukt zur Synthese der Aglykone nach Anspruch 1.

13. Verbindung, deren einer Antipode der allgemeinen Formel XV

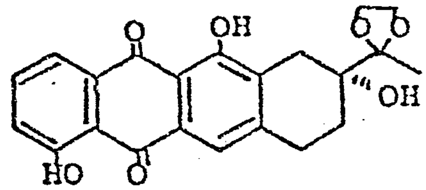

(XV)

entspricht, als Zwischenprodukt zur Synthese der Aglykone nach Anspruch 1.

14. Verbindung, deren einer Antipode der allgemeinen Formel XVI

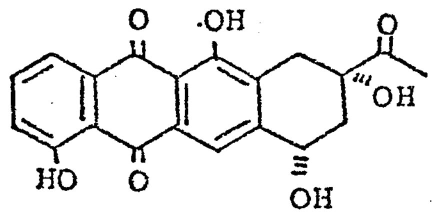

(XVI)

entspricht, als Zwischenprodukt zur Synthese der Aglykone nach Anspruch 1.

15. Verbindung, deren einer Antipode der allgemeinen Formel XVIII

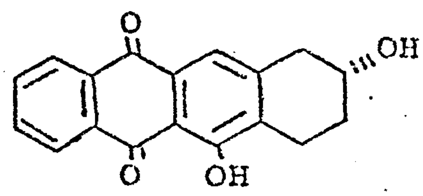

(XVIII)

entspricht, als Zwischenprodukt zur Synthese der Aglykone nach Anspruch 1.

16. Verbindung, deren einer Antipode der allgemeinen Formel XVII

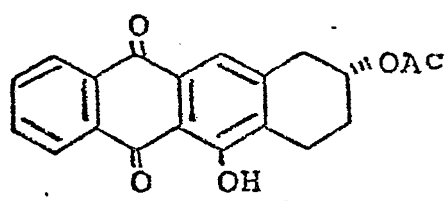

(XVII)

entspricht, als Zwischenprodukt zur Synthese der Aglykone nach Anspruch 1.

17. Verbindung, deren einer Antipode der allgemeinen Formel XIX

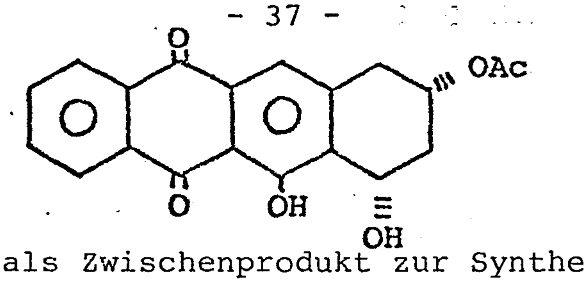

(XIX)

entspricht, als Zwischenprodukt zur Synthese der Aglykone nach Anspruch 1.